# FASCICULE DE BREVET EUROPEEN

(11) **EP 3 057 531 B1**
(45) Date de publication et mention de la délivrance du brevet: **20.07.2022**
(21) Numéro de dépôt: 14799507.0
(22) Date de dépôt: 13.10.2014
(51) Int. Cl.: A61D 19/02

(54) **PAILLETTE POUR LA CONSERVATION D'UNE DOSE PRÉDÉTERMINÉE DE SUBSTANCE À BASE LIQUIDE, NOTAMMENT DE LA SEMENCE ANIMALE PURE OU DILUÉE; ET ENSEMBLE LA COMPORTANT**
BEHÄLTERSTROHHALM ZUM KONSERVIEREN EINER VORBESTIMMTEN DOSIS EINER FLÜSSIGKEITSBASIERTEN SUBSTANZ, INSBESONDERE REINEM ODER VERDÜNNTEM TIERSPERMA ,UND ANORDNUNG DAMIT
CONTAINER STRAW FOR PRESERVING A PREDETERMINED DOSE OF LIQUID-BASED SUBSTANCE, NOTABLY PURE OR DILUTE ANIMAL SEMEN; AND ASSEMBLY COMPRISING THE SAME

(30) Priorité: 15.10.2013 FR 1360036
(43) Date de publication de la demande: 24.08.2016
(73) Titulaire: IMV Technologies, 61300 Saint-Ouen-Sur-Iton (FR)
(72) Inventeur: SCHMITT, Eric, 53700 Villaines-la-Juhel (FR); CARION, Olivier, 83136 La Roquebrussanne (FR); GORGES, Jean-Charles, 72610 Chenay (FR)
(74) Mandataire: Santarelli
(86) Numéro de dépôt international: PCT/FR2014/052597
(87) Numéro de publication internationale: WO 2015/055929

(56) Documents cités:
- FR-A1- 2 824 255
- US-A1- 2006 177 352

## Description

L'invention a trait d'une manière générale à la conservation d'une dose prédéterminée de substance à base liquide, notamment de la semence animale pure ou diluée ; et plus particulièrement aux paillettes pour effectuer une telle conservation.

On sait qu'une telle paillette comporte un tube et un bouchon disposé dans le tube. Le bouchon est habituellement du type tripartite décrit à l'origine dans le brevet français 995.878, correspondant au brevet britannique 669,265, c'est-à-dire formé par deux tampons en substance fibreuse enserrant une poudre se transformant au contact d'un liquide en une pâte ou gel imperméable adhérant à la paroi du tube pour que le bouchon soit étanche aux liquides.

Des bouchons semblables mais perfectionnés sont décrits par les demandes de brevet français 2 824 255 et 2 824 256.

On connait également des bouchons d'un autre type, par exemple un bouchon fait d'un cylindre monobloc de matière microporeuse hydrophobe décrit par la demande de brevet européen 0 873 726 ou un bouchon fait d'un cylindre monobloc de matière microporeuse auto-scellante fritté décrit par la demande PCT WO 2010/070533.

A l'état initial, le bouchon est disposé au voisinage de l'une des extrémités du tube et il est prévu qu'à l'état rempli, la dose de substance liquide qui doit être conservée dans la paillette soit disposée entre le bouchon et l'autre extrémité du tube (extrémité la plus éloignée du bouchon). Le tube et le bouchon sont configurés pour que le bouchon puisse coulisser dans le tube vers l'extrémité initialement la plus éloignée du bouchon.

Pour remplir la paillette, l'extrémité la plus proche du bouchon est mise en communication avec une source de vide tandis que l'extrémité la plus éloignée du tube est mise en communication avec un récipient contenant la substance à introduire dans la paillette.

L'air initialement contenu entre le bouchon et l'extrémité la plus éloignée du tube est aspiré au travers du bouchon tandis que la substance progresse dans le tube jusqu'à ce qu'elle rencontre le bouchon.

Le cas échéant, après remplissage, la paillette est soudée au voisinage de l'une ou de ses deux extrémités et est stockée au froid.

Pour vider la paillette, le cas échéant après découpage des portions d'extrémité soudées et décongélation, on fait pénétrer dans le tube par l'extrémité la plus proche du bouchon une tige qui vient porter contre le bouchon. Avec cette tige, on fait coulisser le bouchon à la façon d'un piston vers l'extrémité la plus éloignée du bouchon, ce qui provoque l'expulsion de la dose de substance qui avait été introduite dans la paillette.

L'invention vise à ce qu'une telle paillette soit capable d'être discriminée d'autres paillettes.

L'invention propose à cet effet un ensemble selon la revendication 1. Des modes de réalisation préférés sont spécifiés dans les revendications dépendantes.

Au contraire, une paillette conventionnelle émet de la lumière dont le spectre est relativement uniforme.

La détection de la présence ou de l'absence, dans la lumière émise par le composant indicateur, du ou des pics ayant une crête de longueur d'onde prédéterminée permet de reconnaître si une paillette dont on est en présence est une paillette ordinaire ou une paillette prévue pour être discriminée.

Une telle détermination est particulièrement simple à opérer, ici de façon automatique par le dispositif de reconnaissance.

La mise en oeuvre de l'ensemble selon l'invention est en outre faisable de façon particulièrement simple et économique, notamment ainsi qu'exposé ci-après.

Selon des caractéristiques avantageuses :
- ledit composant indicateur est configuré pour émettre de la lumière dont le spectre comporte plusieurs dits pics dont les crêtes sont de longueurs d'onde différentes ;
- ledit composant indicateur est configuré pour émettre de la lumière dont le spectre comporte ledit au moins un pic en réponse à un éclairage par de la lumière comportant une plage de longueurs d'onde prédéterminée décalée par rapport à la longueur d'onde prédéterminée de ladite crête ;
- ledit composant indicateur fait partie dudit bouchon et est configuré pour émettre de la lumière comportant ledit au moins un pic lorsqu'il a été en contact avec ladite substance et ne comporte pas ledit au moins un pic en l'absence de contact préalable avec ladite substance ;
- ledit bouchon est formé par deux tampons en substance fibreuse enserrant un agent de scellement formé par une poudre se transformant au contact de ladite substance en une pâte ou gel imperméable adhérant à la paroi du tube pour que le bouchon soit étanche aux liquides, avec ledit composant indicateur qui comporte ledit agent de scellement ;
- ladite poudre comporte de la poudre d'un sel non fluorophore à l'état sec et fluorophore quand il est dissout dans l'eau ;
- ledit sel fait partie du groupe comportant un sel de fluorescéine, un sel de Rhodamine B, un sel de Rhodamine 6G et un sel d'Eriochrome^{®} Cyanine R ;
- ladite poudre comporte entre 1/100 et 1/100000 en poids de ladite poudre de sel ;
- ladite poudre est formée par ladite poudre de sel et par de la poudre de matière polymérisant au contact de l'eau ;
- ladite poudre de matière polymérisant au contact de l'eau est de l'alginate ;
- ledit composant indicateur comporte un fil indicateur configuré pour émettre ladite lumière dont le spectre comporte ledit au moins un pic ;
- ledit bouchon est formé par deux tampons en substance fibreuse tressée enserrant un agent de scellement formé par une poudre se transformant au contact d'un liquide en une pâte ou gel imperméable adhérent à la paroi du tube pour que le bouchon soit étanche aux liquides, avec au moins l'un des deux tampons qui comporte ledit fil indicateur ;
- ledit composant indicateur du bouchon de la paillette est configuré pour émettre de la lumière dont le spectre comporte ledit au moins un pic en réponse à un éclairage par de la lumière comportant une plage de longueurs d'onde prédéterminée décalée par rapport à la longueur d'onde prédéterminée de ladite crête ; et ledit dispositif de reconnaissance comporte en outre un organe d'éclairage pour éclairer ledit composant indicateur par de la lumière comportant ladite plage de longueurs d'onde prédéterminée ; et/ou
- ledit dispositif de reconnaissance est configuré pour détecter l'absence ou la présence de chaque dit pic.

L'exposé de l'invention sera maintenant poursuivi par la description d'exemples de réalisation, donnée ci-après à titre illustratif et non limitatif, en référence aux dessins annexés, sur lesquels :
- la figure 1 est une vue schématique en coupe longitudinale d'une paillette convenant pour faire partie d'un ensemble selon l'invention, à l'état vide ;
- la figure 2 est une vue semblable à la figure 1 mais montrant la paillette à l'état rempli ;
- la figure 3 est un graphe illustrant le spectre de la lumière émise par un agent fluorophore que comporte le bouchon de la paillette lorsque la paillette est à l'état rempli ;
- la figure 4 est une vue semblable à la figure 2 mais montrant la paillette dans une machine comportant un dispositif de reconnaissance de la paillette ;
- la figure 5 est une vue semblable à la figure 4 mais montrant une variante du dispositif de reconnaissance de la paillette ; et
- la figure 6 est une vue semblable à la figure 3 mais pour une variante de l'agent fluorophore.

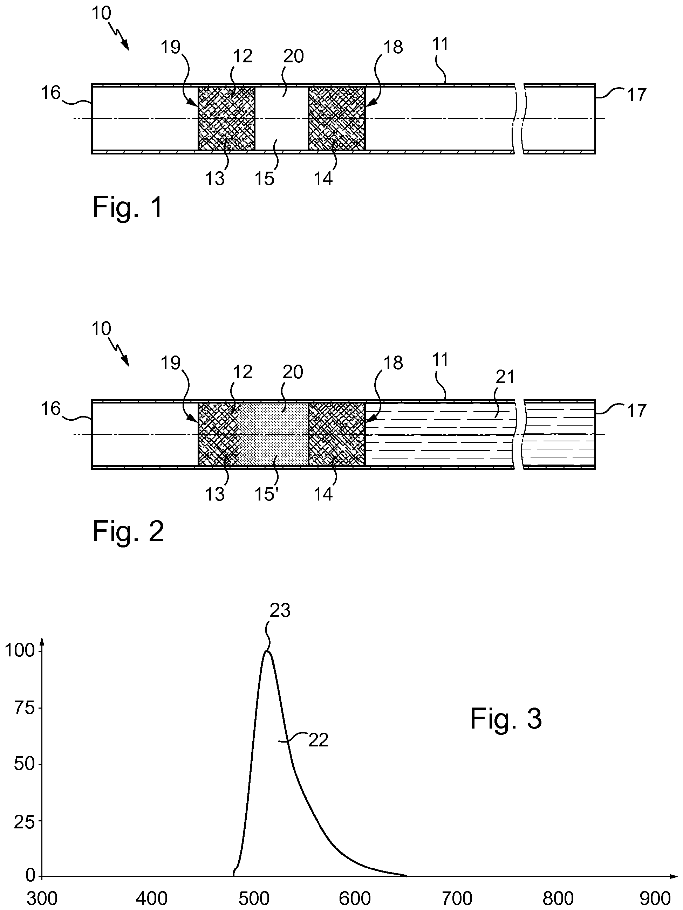

La paillette 10 illustrée sur la figure 1 comporte un tube 11 et un bouchon 12.

Le tube 11 est classiquement en matière plastique extrudée, ici transparente, avec un diamètre interne qui est par exemple de 1,6 ou de 2,5 mm et une longueur de l'ordre de 133 mm.

Le bouchon 12 est du type tripartite, c'est-à-dire formé par deux tampons 13 et 14 d'une substance fibreuse enserrant un agent de scellement 20 formé par une poudre 15 (figure 1) capable de se transformer au contact d'un liquide en une pâte ou gel 15' (figure 2) imperméable adhérant à la paroi du tube 11 pour que le bouchon 12 soit étanche aux liquides.

A l'état initial, illustré sur la figure 1, le bouchon 12 est disposé au voisinage de l'extrémité 16 du tube 11 et il est prévu qu'à l'état rempli, la dose de substance à base liquide qui doit être conservée dans la paillette 10 soit disposée entre le bouchon 12 et l'extrémité 17 du tube 11 la plus éloignée du bouchon 12.

Pour remplir la paillette 10, l'extrémité 16 est mise en communication avec une source de vide tandis que l'extrémité 17 est mise en communication avec un récipient contenant la substance à introduire dans la paillette.

L'air initialement contenu entre le bouchon 12 et l'extrémité 17 est aspiré au travers du bouchon 12 tandis que la substance 21 (figure 2) progresse dans le tube 11 jusqu'à ce qu'elle rencontre le bouchon 12, par l'extrémité 18 de celui-ci tournée vers l'extrémité 17 du tube 11, c'est-à-dire l'extrémité du bouchon 12 que l'on voit à droite sur les figures 1 et 2.

La paillette 10 est alors à l'état rempli montré sur la figure 2.

Le cas échéant, après remplissage, la paillette est soudée au voisinage de l'une ou de ses deux extrémités 16 et 17 et est stockée au froid.

Pour vider la paillette 10, le cas échéant après découpage des portions d'extrémité soudées et décongélation, on fait pénétrer dans le tube 11 une tige qui vient porter contre l'extrémité 19 du bouchon 12 (extrémité située du côté opposé à l'extrémité 18).

Avec cette tige, on fait coulisser le bouchon 12 à la façon d'un piston vers l'extrémité 17 ou l'extrémité correspondante après découpe de la portion soudée, ce qui provoque l'expulsion de la dose de substance 21 qui avait été introduite dans la paillette.

On observera, en comparant les figures 1 et 2, que le bouchon 12 a un aspect différent lorsque la paillette 10 est à l'état vide (figure 1) et à l'état rempli (figure 2).

Lorsque la paillette 10 est à l'état vide, l'agent de scellement 20 (poudre 15) du bouchon 12 a une première couleur et lorsque la paillette 10 est à l'état rempli, l'agent de scellement 20 (gel 15') a une seconde couleur.

Ici, la teinte de la première couleur (état vide) est blanche légèrement marron tandis que la teinte de la seconde couleur (état rempli) est jaune légèrement verte.

Par exemple, la poudre 15, telle que vue au travers du tube 11, a une couleur Pantone^{®} 155U et le gel 15', tel que vu au travers du tube 11, a une couleur Pantone^{®} 395C.

On rappelle ici que la teinte d'une couleur correspond aux longueurs d'onde (ou à la longueur d'onde unique si la couleur fait partie de l'arc-en-ciel) de la lumière émise par l'objet ayant cette couleur. La teinte n'est qu'une des composantes de la couleur, qui dépend d'autres paramètres tels que la luminosité et la saturation.

Le changement de teinte entre la poudre 15 et le gel 15' est dû à la présence, dans l'agent de scellement 20, d'un produit changeant de teinte entre l'état sec et l'état dissout dans l'eau.

Ici, le produit changeant de teinte est le sel sodique de fluorescéine.

On notera que le sel sodique de fluorescéine n'est pas spermicide et convient donc pour le contact avec de la semence animale.

On sait que le sel sodique de fluorescéine a la formule suivante : 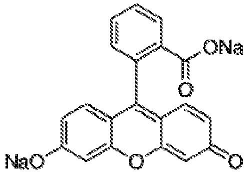 et qu'il est identifié par le numéro CAS 518-47-8.

On sait également que le sel sodique de fluorescéine est un sel fluorofore, c'est-à-dire capable d'émettre de la lumière de fluorescence, lorsqu'il est dissout dans l'eau ; tandis qu'à l'état sec il s'agit d'un sel non fluorofore.

Lorsque l'agent de scellement 20 du bouchon 12 est à l'état sec (poudre 15), le sel sodique de fluorescéine n'émet pas de lumière de fluorescence puisqu'il est à l'état sec. Lorsque l'agent de scellement 20 du bouchon 12 est à l'état humecté (gel 15'), le sel sodique de fluorescéine est dissout dans l'eau contenue dans le gel 15' et émet alors de la lumière de fluorescence.

Le changement de teinte de l'agent de scellement 20 est dû à l'ajout de la lumière de fluorescence.

Grâce à la présence du sel sodique de fluorescéine, l'agent de scellement 20 forme un composant indicateur de contact entre le bouchon 12 et la substance 21 : l'agent de scellement 20 a une première couleur prédéterminée en l'absence de contact préalable avec la substance 21 et une seconde couleur prédéterminée, ayant une teinte différente de la teinte de la première couleur, lorsque l'agent de scellement 20 a été en contact avec la substance 21.

On observera, comme illustré sur la figure 2, qu'à l'état humecté du bouchon 12, une partie du tampon 13 a pris la même teinte que le gel 15'.

En effet, lors du remplissage de la paillette, entre le moment où la substance 21 a atteint la poudre 15 et le moment où la poudre 15 s'est transformée en un gel 15' étanche aux liquides, une petite quantité de poudre 15 dissoute par la substance 21 mais non encore gélifiée a été absorbée par le tampon 13.

Le composant indicateur de contact avec la substance 21 que forme l'agent de scellement 20 est utile pour contrôler le bon remplissage de la paillette 10, et plus précisément la bonne humectation du bouchon 12 par la substance 21.

On sait qu'il est très important, pour la bonne conservation de la dose de substance 21 contenue dans la paillette 10, que le bouchon 12 soit correctement humecté. En effet, dans le cas où la paillette 10 n'est pas soudée à ses extrémités, ou soudée à la seule extrémité 17 la plus éloignée du bouchon 12, l'herméticité de la paillette 10 est assurée en partie par le bouchon 12.

Lors du vidage de la paillette 10, l'humectation correcte du bouchon 12 lors du remplissage permet au bouchon 12 de jouer son rôle de piston sans qu'il y ait des fuites entre le tube 11 et le bouchon 12.

Le contrôle du bon remplissage de la paillette peut s'opérer visuellement par l'opérateur, par simple vérification que l'agent de scellement 20 du bouchon 12 a bien pris la teinte de la seconde couleur prédéterminée, c'est-à-dire une teinte jaune légèrement verte dans le présent exemple.

Le contrôle du bon remplissage de la paillette 10 peut également s'effectuer de façon automatique, comme on va l'expliquer ci-après.

La figure 3 est un graphe illustrant le spectre de la lumière émise par le sel sodique de fluorescéine lorsqu'il est dissout dans l'eau, soit l'agent fluorophore que contient le gel 15'. Sur le graphe de la figure 3, sont portées en abscisses les longueurs d'onde en nm et en ordonnées l'intensité relative d'émission.

On voit que le sel sodique de fluorescéine à l'état dissout dans l'eau émet de la lumière dont le spectre comporte un pic 22 ayant une crête 23 de longueur d'onde de l'ordre de 520 nm, ce qui correspond à la teinte jaune légèrement verte susmentionnée ; et que ce pic est relativement étroit, ce qui est typique de la lumière de fluorescence.

La lumière émise par l'agent de scellement 20 à l'état humecté (gel 15') a un spectre plus étendu mais comporte également le pic 22 ayant la crête 23.

Dans ce spectre, le sommet du pic 22 situé au voisinage de la crête 23 se détache nettement du reste du spectre et peut donc être repéré relativement aisément par un dispositif automatique.

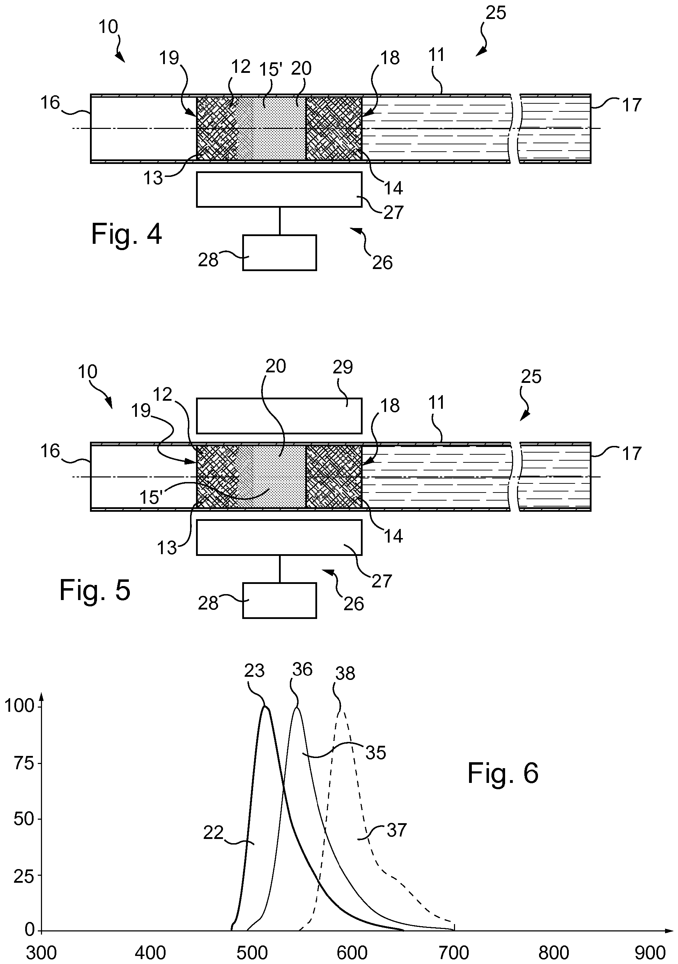

La figure 4 montre une machine 25 de remplissage de paillettes comportant un dispositif 26 de contrôle de remplissage des paillettes 10 remplies par la machine 25.

Le dispositif de contrôle 26 comporte un organe 27 pour détecter si le composant indicateur 20 a pris la teinte de la seconde couleur, c'est-à-dire la teinte du gel 15'.

Ici, l'organe de détection 27 comporte un organe photorécepteur électronique et un organe d'analyse électronique des informations fournies par l'organe photorécepteur pour déterminer si le spectre de la lumière reçue par l'organe photodétecteur comporte le sommet du pic 22 situé au voisinage de la crête 23.

En outre de l'organe de détection 27, le dispositif de contrôle 26 comporte une unité de traitement 28 reliée à l'organe de détection 27 et configurée pour émettre un signal d'arrêt de la machine de remplissage 25 au cas où l'organe de détection 27 détecte une paillette 10 dans laquelle le composant indicateur 20 n'a pas pris la teinte de la seconde couleur.

Lorsque ce signal d'arrêt est émis, la machine de remplissage 25 s'arrête.

Un opérateur peut alors venir identifier les raisons du mauvais remplissage de la paillette et régler l'éventuel problème s'étant produit.

On notera qu'à l'état sec, le sel sodique de fluorescéine est hydrophile. Il se dissout donc quasi-instantanément au contact de la substance 21 à base liquide qui comporte une importante proportion d'eau. En conséquence, le temps de réaction du composant indicateur de contact que forme l'agent de scellement 20 est particulièrement bref.

Grâce à cette vitesse de réaction élevée, le dispositif de remplissage 26 peut être disposé dans la machine 25 au niveau d'un poste de remplissage et détecter quasi-instantanément un défaut de remplissage de paillette.

En arrêtant aussitôt la machine de remplissage, l'on minimise le nombre de paillettes mal remplies, ce qui est très important puisqu'en général la substance à conserver dans une paillette a une valeur économique élevée.

Le temps de réaction rapide permet la mise en œuvre du contrôle de remplissage y compris dans les machines à haute cadence, pouvant remplir jusqu'à plusieurs milliers de paillettes par heure.

Le dispositif de contrôle de remplissage 26 montré sur la figure 4 est capable de fonctionner en éclairage ambiant.

Dans la variante montrée sur la figure 5, utile notamment dans les situations où l'éclairage ambiant est insuffisant, le dispositif de contrôle 26 comporte en outre un organe d'éclairage 29 de l'agent de scellement 20.

On sait que le sel sodique de fluorescéine à l'état dissout dans l'eau est excité principalement par un ensemble de longueurs d'onde situé au voisinage de 495 nm, soit dans une plage de longueurs d'onde prédéterminée décalée par rapport à la longueur d'onde de la crête 23 (520 nm).

L'organe d'éclairage 29 émet de la lumière comportant cette plage de longueurs d'onde prédéterminée.

Il est avantageux, pour éviter que l'organe d'éclairage 29 perturbe l'organe de détection 27, de configurer l'organe d'éclairage 29, en tirant parti du décalage entre la longueur d'onde d'excitation et la longueur d'onde d'émission, pour ne pas émettre de lumière ou en tout cas un minimum de lumière dans la plage de longueurs d'onde d'émission.

Dans l'exemple illustré du bouchon 12, la poudre 15 comporte 1/1000 en poids de poudre de sel sodique de fluorescéine à l'état sec.

Une plage convenant à la mise en œuvre de l'invention est de 1/100 à 1/100000 en poids de poudre de sel sodique de fluorescéine à l'état sec.

Avantageusement, la plage est de 1/500 à 1/50000, et encore plus avantageusement de 1/1000 et 1/25000.

Pour avoir une bonne homogénéité, la poudre 15 est préparée par mélanges successifs.

Dans l'exemple illustré du bouchon 12, la poudre 15 est formée exclusivement par de la poudre de sel sodique de fluorescéine à l'état sec et par de la poudre de matière polymérisant au contact de l'eau.

Ici, la poudre de matière polymérisant au contact de l'eau est de l'alginate.

Dans une variante de l'agent de scellement 20, la poudre de sel sodique de fluorescéine à l'état sec est remplacée par un autre produit non fluorophore à l'état sec et fluorophore quand il est dissout dans l'eau, qui est sous la forme d'un sel à l'état sec.

Il s'agit par exemple d'un autre sel de fluorescéine, d'un sel de Rhodamine B, d'un sel de Rhodamine 6G et/ou d'un sel d'Eriochrome^{®} Cyanine R.

On sait que la Rhodamine B a la formule suivante : 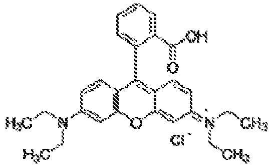 et qu'elle est identifiée par le numéro CAS 81-88-9

On sait que la Rhodamine 6G a la formule suivante : 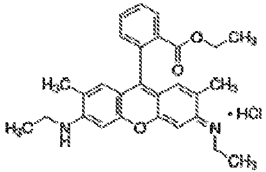 et qu'elle est identifiée par le numéro CAS 989-38-8

On sait que l' Eriochrome^{®} Cyanine R a la formule suivante : 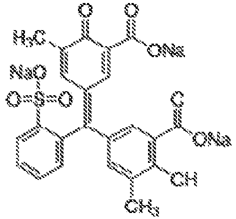 et qu'elle est identifiée par le numéro CAS 64-18-9

La figure 6 montre de la même façon que la figure 3 le spectre de la lumière émise par un mélange de fluorescéine, de Rhodamine 6G et de Rhodamine B.

Ce spectre comporte un pic 35 ayant une crête 36 pour la Rhodamine 6G et un pic 37 ayant une crête 38 pour la Rhodamine B.

On voit que la crête 36 a une longueur d'onde de l'ordre de 553 nm et que la crête 38 a une longueur d'onde de l'ordre de 579 nm.

L'agent fluorophore que comporte l'agent de scellement 20 à l'état humecté (gel 15') peut être formé, comme illustré sur la figure 6, par un mélange de fluorescéine, de Rhodamine 6G et de Rhodamine B ou par un seul ou par deux de ces produits.

En choisissant un ou plusieurs de ces produits, on peut ajuster la teinte de l'agent de scellement 20 à l'état humecté (gel 15'). La sélection de la teinte du composant indicateur que constitue l'agent de scellement 20 lorsqu'il a été en contact avec la substance, permet de reconnaître la paillette 10, visuellement ou par analyse du spectre de la lumière émise, notamment à partir de l'emplacement de la ou des crêtes telles que 23, 36 et 38, choisies pour ne pas être superposées.

La détection de la présence ou de l'absence de chacune des crêtes 23, 36 et 38 permet d'effectuer une reconnaissance de la paillette particulièrement poussée.

On notera qu'en choisissant différents mélanges, il est possible de disposer d'une gamme de paillettes ayant chacune un composant identificateur configuré pour émettre de la lumière dont le spectre comporte une combinaison unique de pics de lumière ayant chacun un emplacement prédéterminé, de sorte que le dispositif de reconnaissance 26 peut également servir à la reconnaissance d'un certain type de paillettes à l'intérieur d'une gamme de paillettes.

Une telle capacité de reconnaissance est particulièrement utile, par exemple pour s'assurer de l'origine de la paillette ou pour s'assurer qu'elle a la nature adéquate pour conserver la semence en cours de remplissage par la machine.

Dans des variantes non illustrées, le dispositif 27 ne sert pas à vérifier le bon remplissage de la paillette 10 mais uniquement à opérer la reconnaissance de paillettes, dans une machine de remplissage ou dans un autre lieu qu'une machine de remplissage.

Dans des variantes non illustrées, le composant indicateur comporte, en outre du ou des agents fluorophores, un agent colorant.

On sait qu'un agent colorant, contrairement à un agent fluorophore, n'émet pas de lumière comportant un pic ayant une crête de longueur d'onde prédéterminée, mais une plage de longueurs d'onde de même intensité relativement étendue.

La combinaison du spectre d'un agent fluorophore et d'un agent colorant peut être utile pour la qualité de la reconnaissance de l'origine de la paillette.

L'agent colorant, sans être flurophore, est par exemple du bleu de méthylène ou de l'a-zurine.

De tels produits colorants, lorsqu'ils sont à l'état sec, par exemple sous la forme d'une faible proportion de l'agent de scellement 20 à l'état sec (poudre 15) n'influent pas ou n'influent que très peu sur la couleur des autres produits formant le composant indicateur, par exemple la poudre d'alginate. En revanche, lorsque le composant indicateur est humecté, le produit colorant communique sa coloration au reste du composant indicateur, par exemple le gel 15'.

Dans des variantes non illustrées, le composant indicateur est différent de l'agent de scellement 20, par exemple un fil indicateur ayant, tout comme l'agent de scellement 20, la capacité d'émettre de la lumière dont le spectre comporte au moins un pic ayant une crête de longueur d'onde prédéterminée. Un tel fil indicateur est associé au bouchon 12 ou à un bouchon de type différent, par exemple un bouchon fait d'un cylindre monobloc tel que décrit dans la demande de brevet européen 0 873 726 ou dans la demande PCT WO 2010/070533.

L'association d'un fil indicateur au bouchon 12 est effectué par exemple, dans le cas où le tampon 13 ou le tampon 14 est une tresse faite de fils de substance fibreuse, en incorporant le fil dans la tresse. Par exemple, le fil indicateur est tressé avec les fils de substance fibreuse.

On peut également associer plusieurs fils indicateurs au bouchon 12.

Par exemple, le ou les fils indicateurs sont disposés au centre du tampon 14 et communiquent leur coloration au reste du tampon 14 lorsqu'il est humecté.

Dans une autre variante, le composant indicateur est formé à la fois par l'agent de scellement 20 et par le fil indicateur associé au bouchon 12.

Dans d'autres variantes, le composant indicateur est différent de l'agent de scellement et d'un fil, par exemple une bille ou une pastille intégrée au bouchon.

Dans d'autres variantes, le changement de couleur du composant indicateur s'effectue au contact d'un liquide autre que l'eau, par exemple un produit contenu dans un dilueur ou un conservateur de semence pour semence animale.

Dans d'autres variantes, la matière du tube tel que 11 n'est pas transparente, mais translucide, par exemple légèrement colorée ; et la détection tient compte du passage de la lumière émise par le composant indicateur au travers de la matière du tube tel que 11.

Dans d'autres variantes, la matière du tube 11 est opaque et le composant indicateur est excité par exemple avec de la lumière infra-rouge ou ultra-violette.

Dans d'autres variantes, le composant indicateur permettant de reconnaître si une paillette dont on est en présence est une paillette ordinaire ou une paillette prévue pour être discriminée, est indépendant du remplissage de la paillette, c'est-à-dire qu'il émet de la lumière de la même façon que la paillette soit à l'état vide ou à l'état rempli. Dans ces variantes, le composant indicateur fait partie ou non du bouchon de la paillette. Par exemple, lorsque le composant indicateur ne fait pas partie du bouchon de la paillette, il s'agit d'un agent fluorophore présent sur un support associé extérieurement au tube 11, voire un agent fluorophore intégré à la matière du tube 11.

De nombreuses autres variantes sont possibles en fonction de circonstances, et l'on rappelle à cet égard que l'invention ne se limite pas aux exemples décrits et représentés.

## Revendications

1. Ensemble comportant une paillette pour la conservation d'une dose prédéterminée de substance à base liquide, comportant un tube (11) et un bouchon (12) perméable aux gaz et étanche aux liquides, lequel bouchon (12) est disposé dans le tube (11) au voisinage d'une extrémité (16), lequel tube (11) et lequel bouchon (12) sont configurés pour que le bouchon (12) puisse coulisser dans le tube (11) vers l'autre extrémité (17), **caractérisé en ce que** ladite paillette (10) comporte un composant indicateur (20) configuré pour émettre, au moins quand la paillette (10) est à l'état rempli, de la lumière dont le spectre comporte au moins un pic (22, 35, 37) ayant une crête (23, 36, 38) de longueur d'onde prédéterminée ; et **en ce que** ledit ensemble comporte en outre un dispositif (26) de reconnaissance de ladite paillette (10), lequel dispositif de reconnaissance (26) comporte un organe de détection (27) pour détecter l'absence ou la présence dudit au moins pic (22, 35, 37) dans la spectre de la lumière émise par ledit composant indicateur (20), lequel dispositif de reconnaissance (26) fait partie d'une machine (25) de remplissage de paillettes et comporte une unité de traitement (28) reliée à l'organe de détection (27) et configurée pour émettre un signal d'arrêt de la machine (25) de remplissage au cas où l'organe de détection (27) détecte une paillette où il y a absence du pic (22, 35, 37) dans le spectre de la lumière émise par le composant indicateur (20).

2. Ensemble selon la revendication 1, **caractérisé en ce que** ledit composant indicateur est configuré pour émettre de la lumière dont le spectre comporte plusieurs dits pics (22, 35, 37) dont les crêtes (23, 36, 38) sont de longueurs d'onde différentes.

3. Ensemble selon l'une quelconque des revendications 1 ou 2, **caractérisé en ce que** ledit composant indicateur (20) est configuré pour émettre de la lumière dont le spectre comporte ledit au moins un pic (22, 35, 37) en réponse à un éclairage par de la lumière comportant une plage de longueurs d'onde prédéterminée décalée par rapport à la longueur d'onde prédéterminée de ladite crête (23, 36, 38).

4. Ensemble selon l'une quelconque des revendications 1 à 3, **caractérisé en ce que** ledit composant indicateur (20) fait partie dudit bouchon (20) et est configuré pour émettre de la lumière comportant ledit au moins un pic (22, 35, 37) lorsqu'il a été en contact avec ladite substance et ne comporte pas ledit au moins un pic (22, 35, 37) en l'absence de contact préalable avec ladite substance.

5. Ensemble selon la revendication 4, **caractérisé en ce que** ledit bouchon (12) est formé par deux tampons (13, 14) en substance fibreuse enserrant un agent de scellement (20) formé par une poudre (15) se transformant au contact de ladite substance (21) en une pâte ou gel (15') imperméable adhérant à la paroi du tube (11) pour que le bouchon (12) soit étanche aux liquides, avec ledit composant indicateur qui comporte ledit agent de scellement (20).

6. Ensemble selon la revendication 5, **caractérisé en ce que** ladite poudre (15) comporte de la poudre d'un sel non fluorophore à l'état sec et fluorophore quand il est dissout dans l'eau.

7. Ensemble selon la revendication 6, **caractérisé en ce que** ledit sel fait partie du groupe comportant un sel de fluorescéine, un sel de Rhodamine B, un sel de Rhodamine 6G et un sel d'Eriochrome^{®} Cyanine R.

8. Ensemble selon l'une quelconque des revendications 6 ou 7, **caractérisé en ce que** ladite poudre (15) comporte entre 1/100 et 1/100000 en poids de ladite poudre de sel.

9. Ensemble selon l'une quelconque des revendications 6 à 8, **caractérisé en ce que** ladite poudre (15) est formée par ladite poudre de sel et par de la poudre de matière polymérisant au contact de l'eau.

10. Ensemble selon la revendication 9, **caractérisé en ce que** ladite poudre de matière polymérisant au contact de l'eau est de l'alginate.

11. Ensemble selon l'une quelconque des revendications 1 à 10, **caractérisé en ce que** ledit composant indicateur comporte un fil indicateur configuré pour émettre ladite lumière dont le spectre comporte ledit au moins un pic (22, 35, 37).

12. Ensemble selon la revendication 11, **caractérisée en ce que** ledit bouchon est formé par deux tampons (13, 14) en substance fibreuse tressée enserrant un agent de scellement (20) formé par une poudre (15) se transformant au contact d'un liquide en une pâte ou gel imperméable (15') adhérent à la paroi du tube pour que le bouchon soit étanche aux liquides, avec au moins l'un des deux tampons (13, 14) qui comporte ledit fil indicateur.

13. Ensemble selon l'une quelconque des revendications 1 à 12, **caractérisé en ce que** le composant indicateur (20) du bouchon (12) de la paillette (10) est configuré pour émettre de la lumière dont le spectre comporte ledit au moins un pic (22, 35, 37) en réponse à un éclairage par de la lumière comportant une plage de longueurs d'onde prédéterminée décalée par rapport à la longueur d'onde prédéterminée de ladite crête (23) ; et ledit dispositif de reconnaissance (26) comporte en outre un organe d'éclairage (29) pour éclairer ledit composant indicateur (20) par de la lumière comportant ladite plage de longueurs d'onde prédéterminée.

14. Ensemble selon l'une quelconque des revendications 1 à 13, **caractérisé en ce que** dispositif de reconnaissance (26) est configuré pour détecter l'absence ou la présence de chaque dit pic (22, 35, 37).

## Patentansprüche

1. Anordnung mit einem Röhrchen zur Aufbewahrung einer vorbestimmten Dosis einer Substanz auf flüssiger Basis, enthaltend einen Rohrabschnitt (11) und einen gasdurchlässigen und flüssigkeitsdichten Stopfen (12), wobei der Stopfen (12) in dem Rohrabschnitt (11) nahe an einem Ende (16) angeordnet ist, wobei der Rohrabschnitt (11) und der Stopfen (12) dazu ausgelegt sind, dass der Stopfen (12) in dem Rohrabschnitt (11) zu dem anderen Ende (17) hin gleiten kann, **dadurch gekennzeichnet, dass** das Röhrchen (10) eine Indikatorkomponente (20) enthält, die dazu ausgelegt ist, zumindest dann, wenn sich das Röhrchen (10) im Füllzustand befindet, Licht zu emittieren, dessen Spektrum zumindest einen Peak (22, 35, 37) mit einem Spitzenwert (23, 36, 38) vorbestimmter Wellenlänge umfasst; und dass die Anordnung ferner eine Erkennungsvorrichtung (26) zum Erkennen des Röhrchens (10) aufweist, wobei die Erkennungsvorrichtung (26) ein Erfassungsorgan (27) aufweist, um das Ausbleiben bzw. Vorliegen des zumindest einen Peaks (22, 35, 37) im Spektrum des von der Indikatorkomponente (20) emittierten Lichts zu erfassen, wobei die Erkennungsvorrichtung (26) zu einer Abfüllmaschine (25) zum Abfüllen von Röhrchen gehört und eine Verarbeitungseinheit (28) aufweist, die mit dem Erfassungsorgan (27) verbunden und dazu ausgelegt ist, ein Signal zum Anhalten der Abfüllmaschine (25) dann auszugeben, wenn das Erfassungsorgan (27) ein Röhrchen erfasst, bei dem der Peak (22, 35, 37) im Spektrum des von der Indikatorkomponente (20) emittierten Lichts ausbleibt.

2. Anordnung nach Anspruch 1, **dadurch gekennzeichnet, dass** die Indikatorkomponente dazu ausgelegt ist, Licht zu emittieren, dessen Spektrum mehrere Peaks (22, 35, 37) mit Spitzenwerten (23, 36, 38) unterschiedlicher Wellenlängen umfasst.

3. Anordnung nach einem der Ansprüche 1 oder 2, **dadurch gekennzeichnet, dass** die Indikatorkomponente (20) dazu ausgelegt ist, Licht zu emittieren, dessen Spektrum den zumindest einen Peak (22, 35, 37) als Reaktion auf eine Beleuchtung mit Licht mit vorbestimmtem Wellenlängenbereich umfasst, der bezüglich der vorbestimmten Wellenlänge des Spitzenwerts (23, 36, 38) verschoben ist.

4. Anordnung nach einem der Ansprüche 1 bis 3, **dadurch gekennzeichnet, dass** die Indikatorkomponente (20) zum Stopfen gehört und dazu ausgelegt ist, Licht zu emittieren, das den zumindest einen Peak (22, 35, 37) dann umfasst, wenn sie mit der Substanz in Kontakt war, und den zumindest einen Peak (22, 35, 37) nicht umfasst, wenn kein vorheriger Kontakt mit der Substanz erfolgt ist.

5. Anordnung nach Anspruch 4, **dadurch gekennzeichnet, dass** der Stopfen (12) aus zwei Puffern (13, 14) aus einer faserförmigen Substanz gebildet ist, die ein Dichtungsmittel (20) umschließen, das aus einem Pulver (15) gebildet ist, das sich bei Kontakt mit der Substanz (21) in eine undurchlässige Paste bzw. ein Gel (15') umwandelt, die bzw. das an der Wand des Rohrabschnitts (11) haftet, damit der Stopfen (12) flüssigkeitsdicht ist, wobei die Indikatorkomponente das Dichtungsmittel (20) aufweist.

6. Anordnung nach Anspruch 5, **dadurch gekennzeichnet, dass** das Pulver (15) Pulver eines Salzes aufweist, das im trockenen Zustand nicht fluoreszierend ist und dann fluoreszierend ist, wenn es in Wasser gelöst ist.

7. Anordnung nach Anspruch 6, **dadurch gekennzeichnet, dass** das Salz zu der Gruppe gehört, die Fluorescein-Salz, Rhodamin-B-Salz, Rhodamin-6G-Salz und Eriochrom ^{®} Cyanin-R-Salz umfasst.

8. Anordnung nach einem der Ansprüche 6 oder 7, **dadurch gekennzeichnet, dass** das Pulver (15) zwischen 1/100 und 1/100000, bezogen auf das Gewicht, des Salzpulvers enthält.

9. Anordnung nach einem der Ansprüche 6 bis 8, **dadurch gekennzeichnet, dass** das Pulver (15) aus dem Salzpulver und aus Pulver aus einem Material, das bei Kontakt mit Wasser polymerisiert, gebildet ist.

10. Anordnung nach Anspruch 9, **dadurch gekennzeichnet, dass** das Pulver aus einem Material, das bei Kontakt mit Wasser polymerisiert, Alginat ist.

11. Anordnung nach einem der Ansprüche 1 bis 10, **dadurch gekennzeichnet, dass** die Indikatorkomponente einen Indikatorfaden aufweist, der dazu ausgelegt ist, das Licht zu emittieren, dessen Spektrum den zumindest einen Peak (22, 35, 37) umfasst.

12. Anordnung nach Anspruch 11, **dadurch gekennzeichnet, dass** der Stopfen aus zwei Stöpseln (13, 14) aus geflochtener, faserförmiger Substanz gebildet ist, die ein Dichtungsmittel (20) umschließen, das aus einem Pulver (15) gebildet ist, das sich bei Kontakt mit einer Flüssigkeit in eine undurchlässige Paste bzw. ein Gel (15') umwandelt, die bzw. das an der Wand des Rohrabschnitts haftet, damit der Stopfen flüssigkeitsdicht ist, wobei zumindest einer der beiden Stöpseln (13, 14) den Indikatorfaden aufweist.

13. Anordnung nach einem der Ansprüche 1 bis 12, **dadurch gekennzeichnet, dass** die Indikatorkomponente (20) des Stopfens (12) des Röhrchens (10) dazu ausgelegt ist, Licht zu emittieren, dessen Spektrum den zumindest einen Peak (22, 35, 37) als Reaktion auf eine Beleuchtung mit Licht mit vorbestimmtem Wellenlängenbereich umfasst, der bezüglich der vorbestimmten Wellenlänge des Spitzenwerts (23) verschoben ist; und die Erkennungsvorrichtung (26) ferner ein Beleuchtungselement (29) zum Beleuchten der Indikatorkomponente (20) mit Licht mit dem vorbestimmten Wellenlängenbereich aufweist.

14. Anordnung nach einem der Ansprüche 1 bis 13, **dadurch gekennzeichnet, dass** die Erkennungsvorrichtung (26) dazu ausgelegt ist, das Ausbleiben bzw. Vorliegen von jedem Peak (22, 35, 37) zu erkennen.

## Claims

1. A set comprising a straw for the preservation of a predetermined dose of liquid-based substance, comprising a tube (11) and a gas-permeable liquid-tight stopper (12), which stopper (12) is disposed in the tube (11) in the neighborhood of one end (16), which tube (11) and which stopper (12) are configured for the stopper (12) to be able to slide in the tube (11) towards the other end (17), **characterized in that** said straw (10) comprises an indicator component (20) configured to emit, at least when the straw (10) is in the filled state, light of which the spectrum comprises at least one peak (22, 35, 37) having a crest (23, 36, 38) of predetermined wavelength; and **in that** said set further comprises a device (26) for recognition of said straw (10), which recognition device (26) comprises a detection member (27) for detecting the absence or the presence of said at least one peak (22, 35, 37) in the light spectrum emitted by said indicator component (20), which recognition device (26) forms part of a machine (25) for filling straws and comprises a processing unit (28) connected to the detection member (27) and configured to emit a stop signal for the filling machine (25) in case the detection member (27) detects a straw in which there is an absence of peak (22,35,37) in the spectrum of light emitted by the indicator component (20).

2. A set according to claim 1, **characterized in that** said indicator component is configured to emit light of which the spectrum comprises several said peaks (22, 35, 37) of which the crests (23, 36, 38) are of different wavelengths.

3. A set according to any one of claims 1 or 2, **characterized in that** said indicator component (20) is configured to emit light of which the spectrum comprises said at least one peak (22, 35, 37) in response to illumination by light comprising a predetermined range of wavelengths that is offset relative to the predetermined wavelength of said crest (23, 36, 38).

4. A set according to any one of claims 1 to 3, **characterized in that** said indicator component (20) forms part of said stopper and is configured to emit light comprising said at least one peak (22, 35, 37) when it has been in contact with said substance and does not comprise said at least one peak (22, 35, 37), in the absence of prior contact with said substance.

5. A set according to claim 4, **characterized in that** said stopper (12) is formed by two plugs (13, 14) made from a fibrous substance enclosing a sealing agent (20) formed by a powder (15) transforming on contact with said substance (21) into an impermeable paste or gel (15') adhering to the wall of the tube (11) so that the stopper (12) is liquid-tight, said indicator component comprising said sealing agent (20).

6. A set according to claim 5, **characterized in that** said powder (15) comprises a powder of a salt that is non-fluorophore in the dry state and fluorophore when it is dissolved in water.

7. A set according to claim 6, **characterized in that** said salt forms part of the group comprising a fluorescein salt, a Rhodamine B salt, a Rhodamine 6G salt and a salt of Eriochrome^{®} Cyanine R.

8. A set according to any one of claims 6 or 7, **characterized in that** said powder (15) comprises between 1/100 and 1/100000 by weight of said salt powder.

9. A set according to any one of claims 6 to 8, **characterized in that** said powder (15) is formed by said salt powder and by powder of material that polymerizes on contact with water.

10. A set according to claim 9, **characterized in that** said powder of material that polymerizes on contact with water is alginate.

11. A set according to any one of claims 1 to 10, **characterized in that** said indicator component comprises an indicator thread configured to emit said light of which the spectrum comprises said at least one peak (22, 35, 37).

12. A set according to claim 11, **characterized in that** said stopper is formed by two plugs (13, 14) made from a braided fibrous substance enclosing a sealing agent (20) formed by a powder (15) which, on contact with a liquid, transforms into an impermeable paste or gel (15') adhering to the wall of the tube so that the stopper is liquid-tight, at least one of the two plugs (13, 14) comprising said indicator thread.

13. A set according to one of claims 1 to 12, **characterized in that** said indicator component (20) of the stopper (12) of the straw (10) is configured to emit light of which the spectrum comprises said at least one peak (22, 35, 37) in response to illumination by light comprising a predetermined range of wavelengths that is offset relative to the predetermined wavelength of said crest (23); and said recognition device (26) further comprises an illuminating member (29) to illuminate said indicator component (20) by light comprising said predetermined range of wavelengths.

14. A set according to any one of claims 1 to 13, **characterized in that** the recognition device (26) is configured to detect the absence or the presence of each said peak (22 35, 37).
